# EUROPEAN PATENT SPECIFICATION

(11) **EP 3 427 169 B1**
(45) Date of publication and mention of the grant of the patent: **11.01.2023**
(21) Application number: 17706517.4
(22) Date of filing: 27.02.2017
(51) Int. Cl.: G16H 40/20, G06Q 10/08, A61M 1/02

(54) **DEVICE FOR BLOOD CONTAINER PROCESSING**
VORRICHTUNG FÜR BLUTBEHÄLTERVERARBEITUNG
DISPOSITIF DESTINÉ AU TRAITEMENT DE CONTENANTS DE SANG

(30) Priority: 09.03.2016 WO PCT/EP2016/159339
(43) Date of publication of application: 16.01.2019
(73) Proprietor: Fresenius Kabi Deutschland GmbH, 61352 Bad Homburg (DE)
(72) Inventor: REMONDI, Fabio, 41037 Mirandola (MO) (IT)
(74) Representative: Maikowski & Ninnemann Patentanwälte Partnerschaft mbB
(86) International application number: PCT/EP2017/054446
(87) International publication number: WO 2017/153191

(56) References cited:
- US-A1- 2008 208 750
- S. GERALD SANDLER ET AL: "Radiofrequency identification technology can standardize and document blood collections and transfusions", TRANSFUSION., vol. 47, no. 5, 1 May 2007 (2007-05-01), pages 763-770, XP055365346, US ISSN: 0041-1132, DOI: 10.1111/j.1537-2995.2007.01188.x
- ZARIC ANDELA ET AL: "RFID-based Smart Blood Stock System [Education Column]", IEEE ANTENNAS AND PROPAGATION MAGAZINE, IEEE SERVICE CENTER, PISCATAWAY, NJ, US, vol. 57, no. 2, 1 April 2015 (2015-04-01), pages 54-65, XP011581402, ISSN: 1045-9243, DOI: 10.1109/MAP.2015.2420491 [retrieved on 2015-05-15]

## Description

### Field of the Invention

The present invention relates to a device for blood container processing, in particular to a MultiBag RFID device and software solution, in particular compatible with Fresenius Kabi's CompoMat G5/CompoMasterNet and CompoGuard/ DonationMasterNet.

### Background of the Invention

RFID technology is becoming important in transfusion technology sector. It is an object to provide a device using RFID, replacing barcode and introducing new possibilities in automatic processing. Interest from market is growing because RFID technology in blood banks and transfusion medicine has the potential to improve operational efficiency and advance patient safety at point of care by automatically identifying, reconciling, and tracking blood products throughout the blood supply chain.

"RFID-based Smart Blood Stock System" by A. Zaric et. al., published in IEEE Antennas and Propagation Magazine (Volume: 57, Issue: 2, April 2015), discloses a multi-drawer RFID-based blood stock system with the ability to read a plurality of blood containers in parallel.

### Summary of the Invention

The invention provides a device for processing a blood container according to the subject matter of the independent claim. Further embodiments are incorporated in the dependent claims.

Accordingly, there is provided a device for blood container processing, wherein the device comprises a receptacle for at least one blood container being equipped with a data storage tag, a data storage reader unit for reading a data storage of a data storage tag on a blood container, a data storage writing unit for writing a data storage of a data storage tag on a blood container, a user interface for inputting blood recipient related information, a data processing unit having a data storage reader interface, a data storage writer interface, an user input interface and a data base communicating interface, wherein the data storage reader unit is communicatively connected to the data storage reader interface, wherein the data storage writer unit is communicatively connected to the data storage writer interface, wherein the user interface is communicatively connected to the user input interface, wherein the data processing unit is communicatively connected to a data base having stored therein blood donator related information, wherein the data storage reading unit is adapted to read an blood donator identifier from a data storage of a blood container, wherein the data processing unit is adapted to request for blood donator related information from an data base based on the read blood donator identifier, wherein the data processing unit is adapted to request an data base for interrelated blood recipient and donator information based on the blood donator related information and the recipient related information input via the user interface, wherein the data processing unit is adapted to control the data storage writing unit to write blood donator related information into the data storage based on the interrelated blood recipient and donator information. Embodiments relating to a device comprising only one receptacle do not fall under the scope of the claims.

This allows a correct processing of all relevant information with respect to the blood donator and the blood recipient. The data base information may be used to match the blood correctly and to avoid any serious injuries on the blood recipient side. If the donator and the recipient are identified, the matching information can be stored on the container. When storing the donator and the recipient, any erroneous use can be avoided.

According to an embodiment, the interrelated blood recipient and donator information is information whether the donator blood matches the recipient.

Thus, the match of the donator and the recipient can directly be stored on the container.

According to an embodiment, the data storage unit is an RFID tag.

Thus, the information can be read contactless and without an energy supply on the container.

According to an embodiment, a data base for interrelated blood recipient and donator information is an external data base and a communicative connection between the device and a data base for interrelated blood recipient and donator information is a wireless connection.

Thus, even complex data volume or computational capacities of external entities may be used. Further, it is possible to use a data base which can be provided form outside with respective information, which can be used by different users, i.e. different devices for blood container processing at different locations.

According to an embodiment, a data base for blood donator related information is an external data base and a communicative connection between the device and a data base for blood donator related information is a wireless connection.

Thus the respective information can be provided even in very flexible situations and locations of the device for blood container processing.

According to an embodiment, the device further comprises a first data base storage unit having stored therein a data base for interrelated blood recipient and donator information.

Thus, the required information can also be provided locally without the need for an external access to a data base. In case the system does not have an external connection to a data base, the system can be protected against external access.

According to an embodiment, the device further comprises a second data base storage unit having stored therein a data base for blood donator related information.

Thus, the required information can also be provided locally without the need for an external access to a data base. In case the system does not have an external connection to a data base, the system can be protected against external access.

According to an embodiment, blood donator related information comprises at least one of the group, the group consisting of donator information, blood separation information, and blood component information.

Thus, relevant information for the compatibility of a donator and the recipient can be provided directly on the container.

The data storage reader unit has at least one data storage reading element and the data storage writer unit has at least one data storage writing element, wherein the data storage reading element and the data storage writing element are combined as a single reading writing unit so as to read and write a combined reading and writing data storage of a data storage tag on a blood container. Embodiments comprising a data storage reader unit having only one data storage reading element and comprising a data storage writer unit having only one data storage writing element do not fall under the scope of the claims.

Thus, a compact reading and writing unit can be provided in order to handle reading and writing information from and to the blood container.

According to an embodiment, the receptacle is adapted for receiving a plurality of blood containers in parallel, wherein the data storage reader unit and the data storage writer unit are adapted to read and write the data storage tags of each of the plurality of blood containers in parallel.

Thus, a parallel processing can be conducted. The several reading and writing elements and/or units can be connected to a bus system so as to communicate with the data processing unit.

According to an embodiment, the device for blood container processing comprises a blood container comprising a volume for receiving blood, a data storage tag, wherein the data storage tag comprises a reading data storage and a writing data storage.

Thus, the related information can directly be provided at the container, so that the information is immediately bound to the container.

According to an embodiment, the data storage tag is an RFID tag.

Thus, a contactless reading and writing can be achieved without the need for a power supply at the container.

According to an embodiment, the reading data storage and the writing data storage are realized as the same chip.

Thus, a compact design an thus a cost efficient chip can be provided.

According to an embodiment, the reading data storage and the writing data storage are realized different chips, wherein the reading chip is adapted to be blocked from being written by the writing mechanism which is used for writing the writing chip.

Thus, a higher data protection can be achieved. Different chips allow a better separation and thus different measures for reading and writing chips.

According to an embodiment, the reading data storage is capable of holding a blood donator identification.

Thus, a unique and optionally un-modifyable donator identification can be achieved.

According to an embodiment, the writing data storage is capable of holding an interrelated blood donator and recipient identification.

Thus, the relevant information can be provided on the container depending on the intended recipient. In case the recipient changes, the updated information can be written onto the writable data storage. As an alternative an "only write once" date storage can be provided, so that any modification can be avoided after writing the recipient relevant and interrelation relevant information onto the container.

### Brief Description of the Drawings

Fig.1 illustrates a collection room with CompoGuard and DonationMasterNet SW.
Fig.2 illustrates an example of 4ple T&B with RCC InLine filter.
Fig.3 illustrates a separation room with CompoMatG5 and CompoMasterNetG5 SW.
Fig. 4 illustrates a device for blood container processing according to an exemplary embodiment of the invention.

### Detailed Description of Exemplary Embodiments of the Invention

In this specification the following abbreviations are used:
- CG:: CompoGuard (Mixing scale)
- G5:: CompoMat G5 (Whole Blood Separator)
- DMNet:: DonationMasterNet (CG Datamanagement)
- CMNetG5:: CompoMasterNet G5 (G5 Datamanagement)
- BBSW:: Blood Bank Software - Hospital or Blood Bank Datamanagement
- MB RFID:: MultiBag RFID Device
- MB SW:: MultiBag RFID Device Datamanagement
- PLT:: Platelets
- BC:: Buffy Coat
- RCC:: Red Cell Concentrate
- DC:: Donation Code
- HC:: Hemocomponent Code

In this specification the following references are used:
- 1: device for blood container processing
- 10: receptacle
- 20: data storage reader unit
- 21: data storage reader interface
- 25: data storage reading element
- 30: data storage writing unit
- 31: data storage writer interface
- 35: data storage writing element
- 40: user interface
- 41: user input interface
- 50: data processing unit
- 60: data communication unit
- 61: data base communicating interface
- 62: first data base storage unit for interrelated blood recipient and donator inform.
- 63: second data base storage unit for blood donator related information
- 162: data base for interrelated blood recipient and donator information
- 163: data base for blood donator related information
- 200: blood container
- 210: volume of container for receiving blood
- 260: data storage tag on container
- 262: reading data storage
- 263: writing data storage

The present invention provides a device for blood container processing. This device at least partially may be implemented as a MultiBag RFID reader-writer device (MB RFID) classified as MD according to 93/42/EEC. The device may further at least partially be implemented as a MultiBag RFID device data management solution (MB SW) being validated verified and documented according the regulatory from FDA according MD software, that will coordinate the read/write function of MB RFID device. The CG and G5 devices and their data management solution DMNet and CMNetG5 respectively, may for example not be modified in any case.

MB RFID may be implemented by a desktop device that is capable to read and write several bags in the same time with <<1% of error for RFID tags LRI 2K 55x55 (inlay 50×50); for further tags% error has to be evaluated but always below 1%. Maximum number of bags (1 bag=1 TAG) may be for example 25 bags. The linear dimensions of the MB RFID where bags can be placed may be for example a A3-format (30cmx42cm LxP). External dimensions of the MB RFID may be designed for example to not exceed 51cm × 65cm × 42cm (LxPxH).

MB RFID may be linked to a personal computer or laptop whereas is installed its data manager software (MB SW). The DMNet and CMNet G5 may be for example installed not in the same PC or laptop, as illustrated in Fig.l and 3.

MB SW may for example be able to communicate with DMNet, CMNet G5 and BB SW. This link may for example be done via serial port and/or USB 2.0/3.0 port and/or wireless protocol (WiFi). Communication between MB RFID and MB SW may be for example be bi-directional. After reading/writing function MB RFID may for example confirm the successful or unsuccessful action. Communication between MB RFID and BB SW may for example be bi-directional. Communication between MB SW and DMNet and CMNetG5 may for example be one-way mode: only pulling data from our DMNet and CMNetG5 toMB SW. MB SW has to pull the ASCII data from DMNet and CMNetG5. At the end of the procedures, DMNet and CMNetG5 may for example create automatically an ASCII file where a constant number of data fields per donation data record is exported. MB SW may for example store in itself these records, making a copy-paste of this file is directly take from DMnet or CMNet, or receiving this file from BB SW. This file for example cannot be deleted or modified from the original folder.

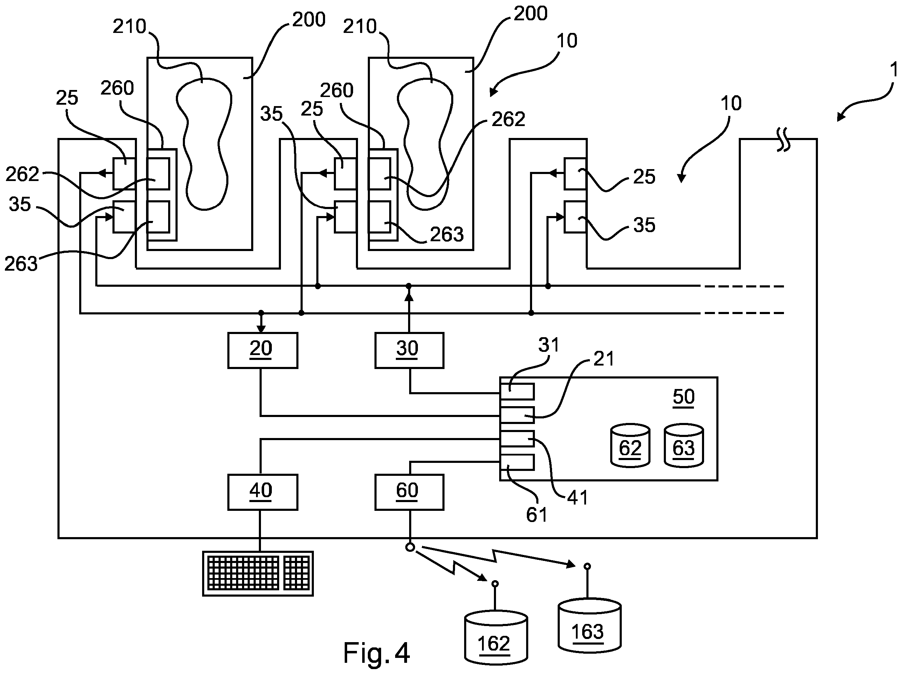

Fig.l illustrates a collection room with CompoGuard and DonationMasterNet SW. The collection room may include seats, referred to as CompoCouch, for blood donators. The donated blood may directly analyzed and monitored. The gained blood may be packed and sealed on site. The blood container may be provided with a unique identifier as well as with a storing tag, which will be described later with respect to Fig. 4. By providing the containers directly with identifiers on site, failures may be reduced in particular with respect to erroneous identification of the blood.

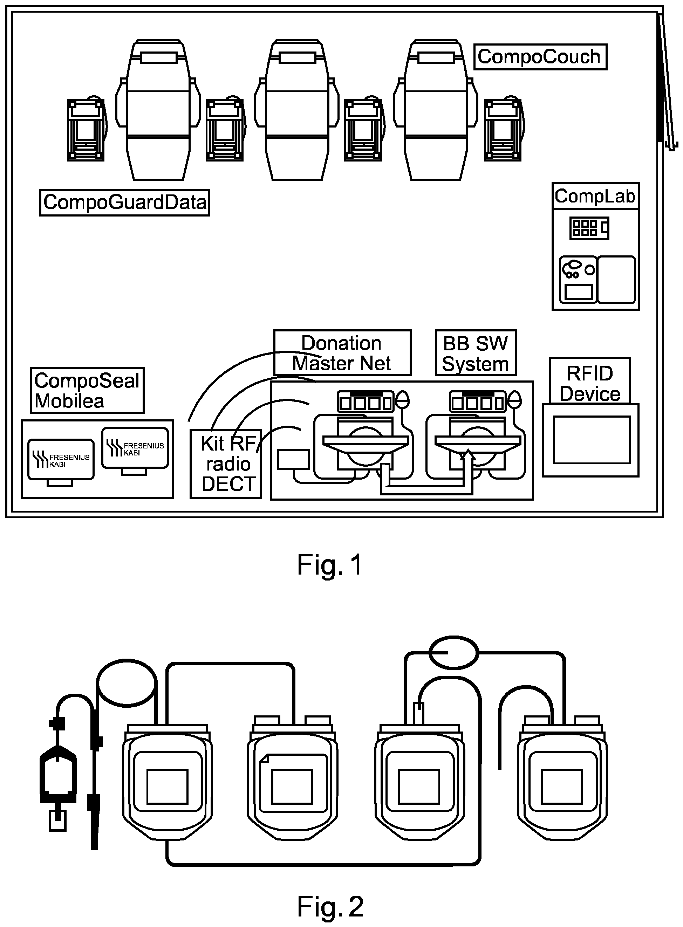

Fig.2 illustrates an example of 4ple T&B with RCC InLine filter. The filter may provide a proper filtering process in order to filter the blood or blood plasma according to the respective requirements.

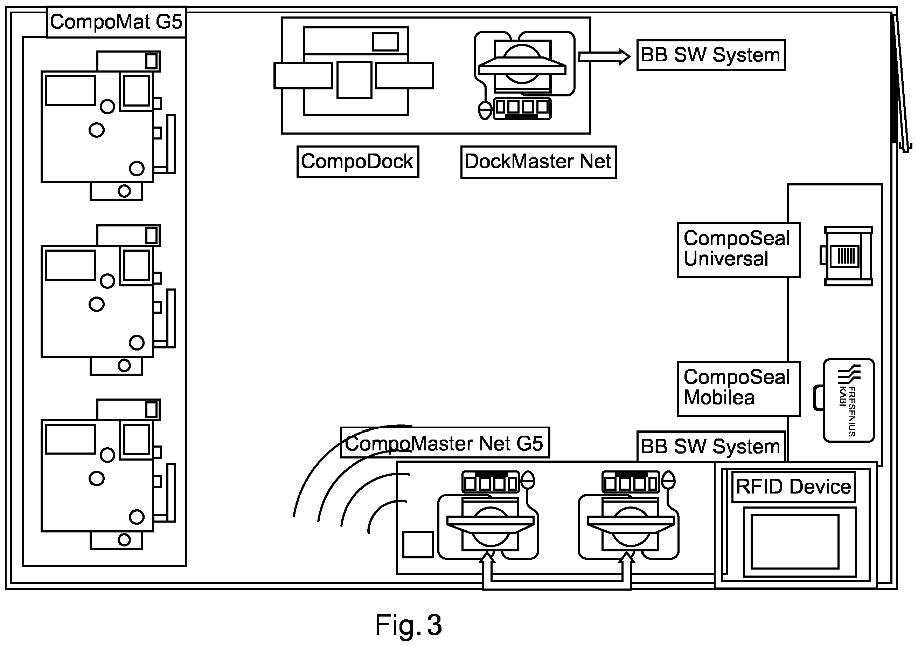

Fig.3 illustrates a separation room with CompoMatG5 and CompoMasterNetG5 SW. The separation room may have all required facilities for a proper processing of the blood and the blood containers.

The workflow in the collection site may for example be as follows:
a. Label are printed in eye-readable barcode and written in RFID (with at least DC and HC) by one printer in connection with BB SW.
b. Bags are labelled (see Fig.2 TAGs mainly on PLS and RCC bag. BC/PRP bag is an option).
c. Donation starts using Fresenius Kabi interface on CG.
d. DMNet receives the list of DC and other information that, in real time, send to all CG present in the network.
e. Simply reading DC, CG set itself on the right program with dedicated collection volume, barcode sequence, alarm settings.
f. At the end of the donation, CG send data into DMNet Software that create automatically an ASCII file where a constant number of 61 data fields per donation data record is exported. The fields are each separated by a semicolon ";". If no data are stored for one field, the field remains empty. The corresponding field length is zero.
g. MB SW stores in itself these records, making a copy-paste of this file that cannot be delete from the original folder because it's required also for the interface between DMNet and BB SW.
h. In MB SW is present a pre-setting phase where, based on the structure of the ASCII file received, is possible define which data has to be write on the TAG. For example interesting data could be only data present in position 6/7/11.
i. When a defined number of bags are positioning on the MB RFID using plastic trays, device reads DC codes written in the TAG and send this list into MB SW.
j. MB SW replies to the device with the corresponding list of data selected as described in phase h per each DC.
   For example: Donation code 202500412536 has this ASCII output file: 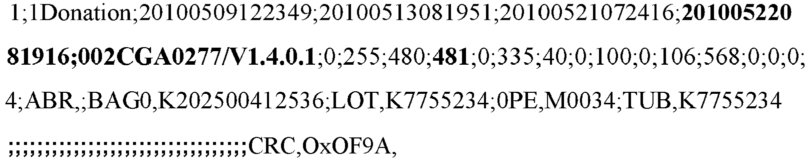 where the position in bold are those selected (position 6/7/11) in phase h.
   So MB SW has to send to MB RFID a string as:
      202500412536, 20100522081916;002CGA0277/V1.4.0.1,481
   This string as to be the same for each DC present on the MB RFID.
k. MB RFID writes in each TAG the correct string received from MB SW.
l. When bags have to be ship to blood bank, shipment list should be made by RFID massive reading, using MB RFID.

For example: Shipment box could be positioned over the MB RFID and the device communicates to MB SW how many and which bags are present.

The workflow in the separation site may for example be as follows:
Fig.2 illustrates a separation room with CompoMatG5 and CompoMasterNetG5 SW:
a. Separation starts using Fresenius Kabi interface on G5.
b. CMNetG5 receives the list of DC that, in real time, send to all G5 present in the network.
c. Simply reading DC, G5 set itself on the right program depending on bag type, donation time, and, i.e. knows if it could produce BC for pool or for waste etc...
d. At the end of the separation, G5 send data into CMNetG5 that create automatically an .csv file where a constant number of 43 data fields per separation data record is exported. The fields are each separated by a semicolon ",". If no data are stored for one field, the field remains empty. The corresponding field length is zero.
   For Example: printout .csv file:
   "DonationBC","DummyBC","7CPT0017","09:19:18","09:20:18","00:01:00"," 09/02/2009","ResultBC","12","TestProgram12","Operator1BC","1","00:00:01" ,"001","002","003","004","005","201","202","203","204","205","1","ProductB C","BatchBC","Operator2BC","CentrifugeBC","AdditionalBC","Incidence1"," Incidence2","Incidence3","Incidence4","IncidenceS","","","101","102","103"," 104","105","09:19:18","09:20:18"
e. MB SW may store in itself these records, making a copy-paste of this file that cannot be delete from the original folder because it's required also for the interface between CMNetG5 and BB SW.
f. In MB SW presents a pre-setting phase where, based on the structure of the .csv file received, should be possible define which Data for which hemocomponent has to be written in the TAG.
   For example interesting data for plasma bag could be only data present in position 7/14 and for RCC bag could be only data in position 7/19.
g. When a defined number of bags are positioning on the MB RFID using plastic trays, device shall read DC and HC codes written in the TAG and send this list into MB SW.
h. MB SW shall reply to the device with the corresponding list of data selected as described in phase f per each DC and HC.
   For example: Code 202500412536 has this .csv output file: 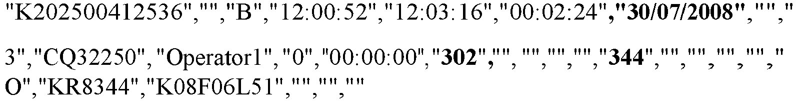 Where the position in bold are those selected (position 7/14/19).
   So MB SW has to send to MB RFID a string as:
      202500412536, 30/07/2008;302;344
   This string as to be the same for each DC present on the MB RFID
i. MB RFID shall write in each TAG the correct string received from MB SW differentiating plasma and RCC bag.
   For example:
   Plasma bag with DC 202500412536 and HC 7 has to receive only 30/07/2008;302. RCC bag with DC 202500412536 and HC 25 has to receive only 30/07/2008;344
j. When bags have to be ship to hospital, shipment list should be made by RFID massive reading, using MB RFID.

For example shipment box could be positioned over the MB RFID and the device should be communicate to MB SW how many and which RCC or PLS bags are present.

For Back-Lab operation, MB RFID may for example able to receive data from BB SW also regarding BC pooling procedures for instance.
a. MB SW should receive a sort of list from BB SW where are included data as pooling barcode, BC barcode assembled, assembling date
   For example:
      I201425236985;202500412536;202500412537;202500412538;202500412539; 202500412540;30/11/2015
   Where in bolt are written the BC barcode.
b. When pooling bag that will contain final PLT pool is positioned over the MB RFID, scanning barcode that identify that bag, data in the list should be written in the TAG previously applied.

Fig. 4 illustrates a schematic buildup of a device according to an embodiment of the invention. The device for blood container processing 1 has one or more receptacles 10 for receiving one or more blood containers 200. A typical bold container 200 has a volume 210 for receiving blood. A typical blood container has a tag 260 for data storage. The tag 260 may have a read section 262 and/or a writing section 263. Both sections can be implemented in a single chip or may be implemented in separate chips. The reading section 262 may contain blood donator related information. This section may be realized as a read only section so that this information cannot be manipulated. The writing section 263 may contain information according to the interrelation between a donator and a recipient. The device 1 in Fig. 4 has a data storage reader unit 20, which may have a reading element 25. This reading element 25 may be arranged so that it can read the donator related information in data storage 262 on the container 200. The device may also have a data storage writing unit 30 with a data writing element 35. The writing element may be adapted for writing information, e.g. of information according to the interrelation between a blood donator and a blood recipient onto a respective storage 263 of the container 200. The device 1 may have a data processing unit 50 having data storage reader interface 21, a data storage writing interface 31, a user input interface 41 and a data base communicating interface 61. The data storage reader interface 21 is connected to the data storage reader 20 and the data storage writing interface 31 is connected to the data storage writer 30. The user input interface 41 may be connected to a user input device 40, which may be a keyboard or any other device for inputting recipient related information. The data base communicating interface 61 may be connected to a data communicating unit 60, which may be adapted for wireless or wire bounded data base connection to an external data base 162 or 163, or to internal data bases 62, 63. The data base storage unit 62 may be for interrelated blood recipient and donator information. The data base storage unit 63 may be for blood donator related information. Accordingly, the data base 162 may be for interrelated blood recipient and donator information, and the data base 163 may be for blood donator related information.

## Claims

1. Device for blood container processing, wherein the device comprises:
a plurality of receptacles (10) for receiving at least one blood container (200), the at least one blood container (200) being equipped with a data storage tag (260),
a data storage reader unit (20) for reading a data storage of a data storage tag on a blood container;
a data storage writing unit (30) for writing a data storage of a data storage tag on a blood container;
wherein the data storage reader unit (20) has a plurality of data storage reading elements (25), wherein each of the plurality of receptacles (10) is associated with one of the plurality of data storage reading elements (25), each data storage reading element (25) being arranged so that it can read donator related information in the data storage of the data storage tag (260) of the at least one blood container (200) received in the associated receptacle (10), and the data storage writing unit (30) has a plurality of data storage writing elements (35), wherein each of the plurality of receptacles (10) is associated with one of the plurality of data storage writing elements (35), wherein the data storage reading element (25) and the data storage writing element (35) associated with the same receptacle (10) are combined as a single reading writing unit so as to read and write a combined reading and writing data storage of a data storage tag (260) on a blood container (200);
a user interface (40) for inputting blood recipient related information;
a data processing unit (50) having a data storage reader interface (21), a data storage writer interface (31), a user input interface (41) and a data base communicating interface (61);
wherein the data storage reader unit (20) is communicatively connected to the data storage reader interface (21);
wherein the data storage writer unit (30) is communicatively connected to the data storage writer interface (31);
wherein the user interface (40) is communicatively connected to the user input interface (41);
wherein the data processing unit (50) is communicatively connected to a data base having stored therein blood donator related information,
wherein the data storage reader unit (20) is adapted to read an blood donator identifier from a data storage of a blood container;
wherein the data processing unit (50) is adapted to request for blood donator related information from an data base (163) based on the read blood donator identifier;
wherein the data processing unit (50) is adapted to request a data base (162) for interrelated blood recipient and donator information based on the blood donator related information and the recipient related information input via the user interface (40),
wherein the data processing unit (50) is adapted to control the data storage writing unit (30) to write blood donator related information into the data storage based on the interrelated blood recipient and donator information.

2. Device for blood container processing according to claim 1, wherein the interrelated blood recipient and donator information is information whether the donator blood matches the recipient.

3. Device for blood container processing according to any one of claims 1 and 2, wherein the data storage tag (260) is an RFID tag.

4. Device for blood container processing according to any one of claims 1 to 3, wherein a data base (162) for interrelated blood recipient and donator information is an external data base and a communicative connection between the device and a data base for interrelated blood recipient and donator information is a wireless connection.

5. Device for blood container processing according to any one of claims 1 to 4, wherein a data base (162) for blood donator related information is an external data base and a communicative connection between the device and a data base for blood donator related information is a wireless connection.

6. Device for blood container processing according to any one of claims 1 to 5, wherein the device further comprises a first data base storage unit (62) having stored therein a data base for interrelated blood recipient and donator information.

7. Device for blood container processing according to claim 6, wherein the device further comprises a second data base storage unit (63) having stored therein a data base for blood donator related information.

8. Device for blood container processing according to any one of claims 1 to 7, wherein blood donator related information comprises at least one of the group, the group consisting of donator information, blood separation information, and blood component information.

9. Device for blood container processing according to any one of claims 1 to 8, wherein the receptacle (10) is adapted for receiving a plurality of blood containers in parallel, wherein the data storage reader unit (20) and the data storage writer unit (30) are adapted to read and write the data storage tags of each of the plurality of blood containers in parallel.

10. Device for blood container processing according to any one of claims 1 to 9, further comprising the at least one blood container (200),
the at least one blood container (200) comprising:
a volume for receiving blood (210);
a data storage tag (260);
wherein the data storage tag comprises a reading data storage (262) and a writing data storage (263).

11. Device for blood container processing according to claim 10, wherein the data storage tag (260) is an RFID tag.

12. Device for blood container processing according to any one of claims 10 and 11, wherein the reading data storage (262) and the writing data storage (263) are realized as the same chip.

13. Device for blood container processing according to any one of claims 10 and 11, wherein the reading data storage (262) and the writing data storage (263) are realized different chips, wherein the reading chip is adapted to be blocked from being written by the writing mechanism which is used for writing the writing chip.

14. Device for blood container processing according to any one of claims 10 to 13, wherein the reading data storage (262) is capable of holding a blood donator identification.

15. Device for blood container processing according to any one of claims 10 to 14, wherein the writing data storage (263) is capable of holding an interrelated blood donator and recipient identification.

## Patentansprüche

1. Vorrichtung für Blutbehälterverarbeitung, wobei die Vorrichtung umfasst:
eine Vielzahl von Aufnahmen (10), um mindestens einen Blutbehälter (200) zu empfangen, wobei der mindestens eine Blutbehälter (200) mit einem Datenspeicher-Tag (260) ausgestattet ist,
eine Datenspeicherlesereinheit (20), um einen Datenspeicher eines Datenspeicher-Tags auf einem Blutbehälter zu lesen;
eine Datenspeicherschreibeinheit (30), um einen Datenspeicher eines Datenspeicher-Tags auf einem Blutbehälter zu schreiben;
wobei die Datenspeicherlesereinheit (20) eine Vielzahl von Datenspeicherleseelementen (25) aufweist, wobei jede von der Vielzahl der Aufnahmen (10) einem von der Vielzahl der Datenspeicherleseelemente (25) zugeordnet ist, jedes Datenspeicherleseelement (25) so angeordnet ist, dass es mit dem Spender zusammenhängende Informationen in dem Datenspeicher des Datenspeicher-Tags (260) des mindestens einen Blutbehälters (200) lesen kann, der in der zugeordneten Aufnahme (10) empfangen wurde, und die Datenspeicherschreibeinheit (30) eine Vielzahl von Datenspeicherschreibelementen (35) aufweist, wobei jede von der Vielzahl der Aufnahmen (10) einem von der Vielzahl der Datenspeicherschreibelemente (35) zugeordnet ist, wobei das Datenspeicherleseelement (25) und das Datenspeicherschreibelement (35), die derselben Aufnahme (10) zugeordnet sind, als einzelne Lese/ Schreib-Einheit kombiniert sind, um so einen kombinierten Lese- und Schreib-Datenspeicher eines Datenspeicher-Tags (260) an einem Blutbehälter (200) zu lesen und darauf zu schreiben;
eine Benutzerschnittstelle (40), um mit dem Blutempfänger zusammenhängende Informationen einzugeben;
eine Datenverarbeitungseinheit (50) mit einer Datenspeicherleserschnittstelle (21), einer Datenspeicherschreiberschnittstelle (31), einer Benutzereingabeschnittstelle (41) und einer Datenbanckommunikationsschnittstelle (61);
wobei die Datenspeicherlesereinheit (20) kommunikativ mit der Datenspeicherleserschnittstelle (21) verbunden ist;
wobei die Datenspeicherschreibereinheit (30) kommunikativ mit der Datenspeicherschreiberschnittstelle (31) verbunden ist;
wobei die Benutzerschnittstelle (40) kommunikativ mit der Benutzereingabeschnittstelle (41) verbunden ist;
wobei die Datenverarbeitungseinheit (50) kommunikativ mit einer Datenbank verbunden ist, in der mit dem Blutspender zusammenhängende Informationen gespeichert sind,
wobei die Datenbanklesereinheit (20) eingerichtet ist, um einen Blutspenderidentifikator aus einem Datenspeicher eines Blutbehälters zu lesen;
wobei die Datenverarbeitungseinheit (50) eingerichtet ist, um mit dem Blutspender zusammenhängende Informationen aus einer Datenbank (163) basierend auf dem gelesenen Blutspenderidentifikator anzufordern;
wobei die Datenverarbeitungseinheit (50) eingerichtet ist, um von einer Datenbank (162) mittels der Benutzerschnittstelle (40) miteinander in Beziehung stehende Blutempfänger- und - spenderinformationen basierend auf den mit dem Blutspender zusammenhängenden Informationen und mit dem Empfänger zusammenhängenden Informationen anzufordern,
wobei die Datenverarbeitungseinheit (50) eingerichtet ist, um die Datenspeicherschreibeinheit (30) so zu steuern, dass basierend auf miteinander in Beziehung stehenden Blutempfänger- und -spenderinformationen mit dem Blutspender zusammenhängende Informationen in den Datenspeicher geschrieben werden.

2. Vorrichtung für Blutbehälterverarbeitung nach Anspruch 1, wobei die in Beziehung stehenden Blutempfänger- und -spenderinformationen Informationen sind, ob das Spenderblut zu dem Empfänger passt.

3. Vorrichtung für Blutbehälterverarbeitung nach einem der Ansprüche 1 und 2, wobei das Datenspeicher-Tag (260) ein RFID-Tag ist.

4. Vorrichtung für Blutbehälterverarbeitung nach einem der Ansprüche 1 bis 3, wobei eine Datenbank (162) für miteinander in Beziehung stehende Blutempfänger- und -spenderinformationen eine externe Datenbank ist, und eine kommunikative Verbindung zwischen der Vorrichtung und einer Datenbank für miteinander in Beziehung stehende Blutempfänger- und - spenderinformationen eine drahtlose Verbindung ist.

5. Vorrichtung für Blutbehälterverarbeitung nach einem der Ansprüche 1 bis 4, wobei eine Datenbank (162) für mit dem Blutspender zusammenhängende Informationen eine externe Datenbank ist, und eine kommunikative Verbindung zwischen der Vorrichtung und einer Datenbank für mit dem Blutspender zusammenhängende Informationen eine drahtlose Verbindung ist.

6. Vorrichtung für Blutbehälterverarbeitung nach einem der Ansprüche 1 bis 5, wobei die Vorrichtung des Weiteren eine erste Datenbankspeichereinheit (62) umfasst, in der eine Datenbank für miteinander in Beziehung stehende Blutempfänger- und -spenderinformationen gespeichert ist.

7. Vorrichtung für Blutbehälterverarbeitung nach Anspruch 6, wobei die Vorrichtung des Weiteren eine zweite Datenbankspeichereinheit (63) umfasst, in der eine Datenbank für mit dem Blutspender zusammenhängende Informationen gespeichert ist.

8. Vorrichtung für Blutbehälterverarbeitung nach einem der Ansprüche 1 bis 7, wobei mit dem Blutspender zusammenhängende Informationen mindestens eines von der Gruppe umfassen, wobei die Gruppe aus Spenderinformationen, Bluttrennungsinformationen und Blutkomponenteninformationen besteht.

9. Vorrichtung für Blutbehälterverarbeitung nach einem der Ansprüche 1 bis 8, wobei die Aufnahme (10) eingerichtet ist, um eine Vielzahl von Blutbehältern parallel zu empfangen, wobei die Datenspeicherlesereinheit (20) und die Datenspeicherschreibereinheit (30) eingerichtet sind, um die Datenspeicher-Tags von jedem von der Vielzahl der Blutbehälter parallel zu lesen und darauf zu schreiben.

10. Vorrichtung für Blutverarbeitung nach einem der Ansprüche 1 bis 9, des Weiteren umfassend den mindestens einen Blutbehälter (200),
wobei der mindestens eine Blutbehälter (200) umfasst:
ein Volumen zum Empfangen von Blut (210);
ein Datenspeicher-Tag (260);
wobei das Datenspeicher-Tag einen Lesedatenspeicher (262) und einen Schreibdatenspeicher (263) umfasst.

11. Vorrichtung für Blutbehälterverarbeitung nach Anspruch 10, wobei das Datenspeicher-Tag (260) ein RFID-Tag ist.

12. Vorrichtung für Blutbehälterverarbeitung nach einem der Ansprüche 10 und 11, wobei der Lesedatenspeicher (262) und der Schreibdatenspeicher (263) als derselbe Chip realisiert sind.

13. Vorrichtung für Blutbehälterverarbeitung nach einem der Ansprüche 10 bis 11, wobei der Lesedatenspeicher (262) und der Schreibdatenspeicher (263) als unterschiedliche Chips realisiert sind, wobei der Lesechip eingerichtet ist, um durch den Schreibmechanismus, der zum Schreiben auf den Schreibchip verwendet wird, so blockiert zu werden, dass er nicht beschrieben werden kann.

14. Vorrichtung für Blutbehälterverarbeitung nach einem der Ansprüche 10 bis 13, wobei der Lesedatenspeicher (262) in der Lage ist, eine Blutspenderidentifikation zu enthalten.

15. Vorrichtung für Blutbehälterverarbeitung nach einem der Ansprüche 10 bis 14, wobei der Schreibdatenspeicher (263) in der Lage ist, eine in Beziehung stehende Blutspender- und -empfängeridentifikation zu enthalten.

## Revendications

1. Dispositif de traitement de contenants de sang, le dispositif comprenant :
une pluralité de réceptacles (10) pour recevoir au moins un contenant de sang (200), l'au moins un contenant de sang (200) étant équipé d'une étiquette de stockage de données (260),
une unité de lecture de stockage de données (20) pour lire un stockage de données d'une étiquette de stockage de données sur un contenant de sang ;
une unité d'écriture de stockage de données (30) pour écrire un stockage de données d'une étiquette de stockage de données sur un contenant de sang ;
l'unité de lecture de stockage de données (20) ayant une pluralité d'éléments de lecture de stockage de données (25), chacun de la pluralité de réceptacle (10) étant associé à l'un de la pluralité d'éléments de lecture de stockage de données (25), chaque élément de lecture de stockage de données (25) étant agencé de sorte qu'il puisse lire des informations liées au donneur dans le stockage de données de l'étiquette de stockage de données (260) de l'au moins un contenant de sang (200) reçu dans le réceptacle associé (10), et l'unité d'écriture de stockage de données (30) ayant une pluralité d'éléments d'écriture de stockage de données (35), chacun de la pluralité de réceptacles (10) étant associé à l'un de la pluralité d'éléments d'écriture de stockage de données (35), l'élément de lecture de stockage de données (25) et l'élément d'écriture de stockage de données (35) associés au même réceptacle (10) étant combinés en tant qu'unité de lecture et d'écriture unique afin de lire et d'écrire un stockage de données de lecture et d'écriture combiné d'une étiquette de stockage de données (260) sur un contenant de sang (200) ;
une interface utilisateur (40) pour entrer des informations liées au receveur de sang ;
une unité de traitement de données (50) ayant une interface de lecture de stockage de données (21), une interface d'écriture de stockage de données (31), une interface d'entrée utilisateur (41) et une interface de communication de base de données (61) ;
l'unité de lecture de stockage de données (20) étant connectée de manière communicative à l'interface de lecture de stockage de données (21) ;
l'unité d'écriture de stockage de données (30) étant connectée de manière communicative à l'interface d'écriture de stockage de données (31) ;
l'interface utilisateur (40) étant connectée de manière communicative à l'interface d'entrée utilisateur (41) ;
l'unité de traitement de données (50) étant connectée de manière communicative à une base de données dans laquelle sont stockées des informations liées aux donneurs de sang,
l'unité de lecture de stockage de données (20) étant adaptée pour lire un identifiant de donneur de sang à partir d'un stockage de données d'un contenant de sang ;
l'unité de traitement de données (50) étant adaptée pour demander des informations liées au donneur de sang à partir d'une base de données (163) sur la base de l'identifiant de donneur de sang lu ;
l'unité de traitement de données (50) étant adaptée pour demander à une base de données (162) des informations de receveur et de donneur de sang interreliées sur la base des informations liées au donneur de sang et des informations liées au receveur entrées par l'intermédiaire de l'interface utilisateur (40),
l'unité de traitement de données (50) étant adaptée pour commander l'unité d'écriture de stockage de données (30) pour écrire des informations liées au donneur de sang dans le stockage de données sur la base des informations de receveur et de donneur de sang interreliées.

2. Dispositif de traitement de contenants de sang selon la revendication 1, dans lequel les informations de receveur et de donneur de sang interreliées sont des informations indiquant si le sang du donneur correspond au receveur.

3. Dispositif de traitement de contenants de sang selon l'une quelconque des revendications 1 et 2, dans lequel l'étiquette de stockage de données (260) est une étiquette RFID.

4. Dispositif de traitement de contenants de sang selon l'une quelconque des revendications 1 à 3, dans lequel une base de données (162) pour des informations de receveur et de donneur de sang interreliées est une base de données externe et une connexion de communication entre le dispositif et une base de données pour des informations de receveur et de donneur de sang interreliées est une connexion sans fil.

5. Dispositif de traitement de contenants de sang selon l'une quelconque des revendications 1 à 4, dans lequel une base de données (162) pour les informations liées aux donneurs de sang est une base de données externe et une connexion de communication entre le dispositif et une base de données pour les informations liées aux donneurs de sang est une connexion sans fil.

6. Dispositif de traitement de contenants de sang selon l'une quelconque des revendications 1 à 5, le dispositif comprenant en outre une première unité de stockage de base de données (62) dans laquelle est stockée une base de données pour des informations de receveur et de donneur de sang interreliées.

7. Dispositif de traitement de contenants de sang selon la revendication 6, le dispositif comprenant en outre une deuxième unité de stockage de base de données (63) dans laquelle est stockée une base de données pour des informations liées au donneur de sang.

8. Dispositif de traitement de contenants de sang selon l'une quelconque des revendications 1 à 7, dans lequel les informations liées au donneur de sang comprennent au moins des informations du groupe, le groupe étant constitué d'informations du donneur, d'informations de la séparation du sang et d'informations des composants sanguins.

9. Dispositif de traitement de contenants de sang selon l'une quelconque des revendications 1 à 8, dans lequel le réceptacle (10) est adapté pour recevoir une pluralité de contenants de sang en parallèle, l'unité de lecture de stockage de données (20) et l'unité d'écriture de stockage de données (30) étant adaptées pour lire et écrire les étiquettes de stockage de données de chacun de la pluralité de contenants de sang en parallèle.

10. Dispositif de traitement de contenants de sang selon l'une quelconque des revendications 1 à 9, comprenant en outre l'au moins un contenant de sang (200), l'au moins un contenant de sang (200) comprenant :
un volume destiné à recevoir du sang (210) ;
une étiquette de stockage de données (260) ;
l'étiquette de stockage de données comprenant un stockage de données de lecture (262) et un stockage de données d'écriture (263).

11. Dispositif de traitement de contenants de sang selon la revendication 10, dans lequel l'étiquette de stockage de données (260) est une étiquette RFID.

12. Dispositif de traitement de contenants de sang selon l'une quelconque des revendications 10 et 11, dans lequel le stockage de données de lecture (262) et le stockage de données d'écriture (263) sont réalisés sous la forme d'une même puce.

13. Dispositif de traitement de contenants de sang selon l'une quelconque des revendications 10 et 11, dans lequel le stockage de données de lecture (262) et le stockage de données d'écriture (263) sont réalisés sous forme de puces différentes, la puce de lecture étant adaptée pour être bloquée contre l'écriture par le mécanisme d'écriture qui est utilisé pour écrire sur la puce d'écriture.

14. Dispositif de traitement de contenants de sang selon l'une quelconque des revendications 10 à 13, dans lequel le stockage de données de lecture (262) est capable de contenir une identification du donneur de sang.

15. Dispositif de traitement de contenants de sang selon l'une quelconque des revendications 10 à 14, dans lequel le stockage de données d'écriture (263) est capable de contenir une identification du donneur et du receveur de sang interreliée.
